# EUROPEAN PATENT SPECIFICATION

(11) **EP 3 054 961 B1**
(45) Date of publication and mention of the grant of the patent: **26.08.2020**
(21) Application number: 14851547.1
(22) Date of filing: 07.10.2014
(51) Int. Cl.: A61K 35/51, A61K 35/28, A61P 37/00, A61K 38/19

(54) **ACTIVATION OF HEMATOPOIETIC PROGENITORS BY PRETRANSPLANT EXPOSURE TO DEATH LIGANDS**
AKTIVIERUNG VON HÄMATOPOIETISCHEN VORLÄUFERN DURCH PRÄTRANSPLANT-AUSSETZUNG GEGEN TODESLIGANDEN
ACTIVATION DE PROGÉNITEURS HÉMATOPOIÉTIQUES PAR EXPOSITION AVANT LA GREFFE AUX LIGANDS DE MORT

(30) Priority: 09.10.2013 US 201361888713 P
(43) Date of publication of application: 17.08.2016
(73) Proprietor: Cellect Biotherapeutics Ltd., Kfar Saba 4442510 (IL)
(72) Inventor: YARKONI, Shai, 4439529 Kfar Saba (IL)
(74) Representative: Vossius & Partner Patentanwälte Rechtsanwälte mbB
(86) International application number: PCT/IL2014/050887
(87) International publication number: WO 2015/052716

(56) References cited:
- WO-A1-2013/132477
- WO-A2-2006/113881
- WO-A2-2012/011113
- MIZRAHI, K. ET AL.: 'Regulatory functions of TRAIL in hematopoietic progenitors: human umbilical cord blood and murine bone marrow transplantation.' LEUKEMIA vol. 24, no. 7, 2010, pages 1325 - 1334, XP055335045 Retrieved from the Internet: <URL:http:// www. nature .com/leu/journal/v24/n7/full/leu201097a.htm l> [retrieved on 2015-01-22]
- PEARL-YAFE, MICHAL ET AL.: 'Fas transduces dual apoptotic and trophic signals in hematopoietic progenitors.' STEM CELLS vol. 25, no. 12, 2007, pages 3194 - 3203, XP009096037 Retrieved from the Internet: <URL:http://onlinelibrary. wiley .com/doi/10.1634/ stemcells.2007-0402/abstract> [retrieved on 2015-01-26]
- MIZRAHI, K. ET AL.: 'Apoptotic signaling through Fas and TNF receptors ameliorates GvHD in mobilized peripheral blood grafts.' BONE MARROW TRANSPLANTATION 2014, XP055229409 Retrieved from the Internet: <URL:http://www. nature .com/bmt/journal/v49/n5/full/bmt201412a.htm l> [retrieved on 2014-02-24]
- MIZRAHI, KEREN ET AL.: 'Activation and crosstalk between TNF family receptors in umbilical cord blood cells is not responsible for loss of engraftment capacity following culture.' AMERICAN JOURNAL OF STEM CELLS vol. 2, no. 3, 2013, page 155, XP055335046 Retrieved from the Internet: <URL:http://www.ncbi.nlm.nih.gov/ pmc/articles/PMC3 875276> [retrieved on 2013-12-22]

## Description

### BACKGROUND OF THE INVENTION

Hematopoietic stem and progenitor cell transplants are a conventional therapeutic modality in oncology, endowed with curative capacity in a variety of radiochemotherapy-resistant malignancies. Improvements and progressive advances in the transplant procedure open the way to wide implementation of hematopoietic transplants in non-oncological disorders, including congenital deficits, inborn and acquired immune deficiency syndromes, aberrant immunity, autoimmune diseases and induction of tolerance to organ and tissue grafts.

The TNF family includes 19 known receptor/ligand interactions, which share a common activity of induction of apoptosis in somatic cells.

Work performed in models of stress hematopoiesis and transplantation using murine and human cells has yielded controversial evidence and interpretation of the role of tumor necrosis factor (TNF) family receptors as dual mediators of apoptosis and stimulation **[1-4**]. On the one hand, the traditional concept has been dominated by the role of these receptors as mediators of apoptosis, one of the few irreversible events in cell development. Accordingly, the Fas/FasL interaction is considered to be a negative regulator of developing hematopoietic progenitors, similar to suppression of clonal expansion in the distal stages of differentiation **[5-7**]. This concept is based on data showing that the Fas receptor suppresses hematopoietic progenitor function *in vitro* **[8, 9],** represses clonogenic activity **[10, 11**], and impairs human cell engraftment in immunocompromized mice **[12,13**] and humans **[11**].

It has been suggested that progenitors are protected from apoptosis due to low levels of expression of receptors of the TNF family, which transduce apoptotic and suppressive signals **[8-10**].

On the other hand, acute upregulation of several TNF family receptors is observed in donor cells soon after homing to and seeding in the recipient bone marrow.

Resistance of hematopoietic progenitors to apoptosis has several significant implications in the transplant setting, as described in WO2007/138597. In one of its aspects, WO2007/138597 proposes to use apoptotic challenge for enrichment of progenitors for transplantation based on a functional characteristic as a substitute for phenotype-based selection. This effective and reliable selection procedure is simple and cheap, and includes in the donor graft the progenitors that express low levels or do not express markers such as CD34 and CD133. In addition, apoptosis-based cell preparation has the distinct advantage of eliminating graft versus host (GvHD) effectors and autoreactive T cells, and malignant cells from the donor graft [33, 34].

The inventors have previously shown that the negative impact of TNF family receptors on hematopoietic cell function *in vitro* is restricted to cytokine-stimulated bone marrow and UCB cells **[8-20,23,27**], which is further accentuated by cytokine withdrawal [**35**]. The fraction of CD34⁺ progenitors expressing Fas display excessive sensitivity to spontaneous apoptosis during extended *ex vivo* culture associated with progressive loss of engraftment potential [**12,13**], however this phenomenon is independent of Fas receptor cross-linking [**28,31**]. It was shown that the inherent progenitor resistance to apoptosis persists *in vivo* after transplantation, prioritizing the apoptosis-insensitive progenitors for engraftment within the hash bone marrow environment caused by injury inflicted by pretransplant conditioning [**28**]. This characteristic of progenitors makes them effective substitutes of immune cells for immunomodulation to alleviate host versus graft (HvG) alloimmune responses that mediate rejection *in vivo,* attained by ectopic expression of membranous FasL protein **[36,37**].

### SUMMARY OF THE INVENTION

The present invention concerns the improvement of hematopoietic cell engraftment by activation of TNF family receptors. In contrast to the traditional concept that activation of death receptors is detrimental to hematopoietic progenitor engraftment and function, the inventors demonstrate inductive activity of these receptors in progenitors that are inherently resistant to receptor-mediated apoptosis. Exposure to Fas-ligand, TNF-α and TRAIL and their combinations *ex vivo* enhance quantitative xenochimerism in SCID mice and improve myeloid reconstitution *in vivo.* Induction of myeloid progenitor activity is also demonstrated in culture. Detailed examples are shown for fresh umbilical cord blood and cryopreserved mobilized peripheral blood, to demonstrate that activation of hematopoietic progenitors encompasses the prevalent sources of donor cell grafts and is independent of storage. The times and concentrations of ligands required for activation of progenitors from various sources vary and have been optimized accordingly. According to the invention, donor hematopoietic grafts are exposed to ligands of the TNF superfamily for progenitor activation to achieve superior quantitative and qualitative engraftment and reconstitution.

Therefore, the present invention relates to the embodiments as characterized in the claims. Thus, it relates to the following items.
1. An ex vivo method for obtaining a population of stem and progenitor cells (SPC) with enhanced engraftment characteristics by activation of TNF family receptors, wherein said cells are suitable for being transplanted into a recipient in need of SPC transplantation, comprising:
   contacting ex vivo a biological sample obtained from a donor, said biological sample comprising a population of SPC, with at least one member of the TNF superfamily or any fragment or derivative thereof which retains the activity of the complete protein, wherein said enhanced engraftment characteristics comprise increasing myeloid, lymphoid, thrombocytic or erythroid reconstitution or activity, wherein the at least one member of the TNF super family is selected from the group consisting of Fas ligand (FasL) and tumor necrosis factor α (TNF-α), and
   wherein said biological sample is umbilical cord blood (UCB) and said contacting of step (a) is for between 18 hours and 48 hours; or
   wherein said biological sample is mobilized peripheral blood (mPB) and said contacting of step (a) is for between 3 hours and 18 hours.
2. The method according to item 1 wherein said SPC are hematopoietic stem and progenitor cells (HSPC).
3. The method according to item 1 wherein said TNF-α is at a concentration of between 10ng/ml and 20ng/ml, or
   wherein said FasL is at a concentration of between 10ng/ml and 50ng/ml.
4. The method according to any one of the preceding items wherein said at least one member of the TNF super family is conjugated to a surface, wherein optionally said surface is a bead, and optionally said conjugation is via a linker.
5. A population of cells for use in a method of enhancing the engraftment of stem cell progenitor cells (SPC) in a recipient in need of SPC transplantation, wherein said cells are stem and progenitor cells (SPC) with enhanced engraftment characteristics wherein said population of SPC with enhanced engraftment characteristics is obtained by activation of TNF family receptors and by contacting ex vivo a biological sample obtained from a donor, said biological sample comprising a population of SPC, with at least one member of the TNF super family or any fragment or derivative thereof which retains the activity of the complete protein, wherein said enhanced engraftment characteristics comprise increasing myeloid, lymphoid, thrombocytic or erythroid reconstitution or activity, wherein the at least one member of the TNF super family is selected from the group consisting of Fas ligand (FasL) and tumor necrosis factor α (TNF-α), and wherein said biological sample is umbilical cord blood (UCB) and said contacting is for between 18 hours and 48 hours; or
   wherein said biological sample is mPB and said contacting of is for between 3 hours and 18 hours.
6. The population of cells according to item 5 wherein said SPC are hematopoietic stem and progenitor cells (HSPC).
7. The population of cells according to item 5, wherein said TNF-α is at a concentration of between 10ng/ml and 20ng/ml, or
   wherein said FasL is at a concentration of between 10ng/ml and 50ng/ml.
8. The population of cells according to any one of items 5-7 wherein said at least one member of the TNF super family is conjugated to a surface, wherein optionally said surface is a bead, and optionally said conjugation is via a linker.

Therefore, in a first of its aspects, there is disclosed a method for enhancing the engraftment of stem and progenitor cells (SPC) in a recipient in need of SPC transplantation, comprising:
a. contacting *ex vivo* a biological sample obtained from a donor, said biological sample comprising a population of SPC, with at least one member of the TNF super family or any fragment or derivative thereof; and
b. after said contacting step (a), transplanting said SPC into said recipient; wherein the transplanted SPC exhibit enhanced engraftment.

In another aspect, there is disclosed a population of cells for use in a method of transplantation, wherein said cells are stem and progenitor cells (SPC) with enhanced engraftment characteristics wherein said population of SPC with enhanced engraftment characteristics is obtained by contacting ex vivo a biological sample obtained from a donor, said biological sample comprising a population of SPC, with at least one member of the TNF super family or any fragment or derivative thereof.

### DESCRIPTION OF THE DRAWINGS

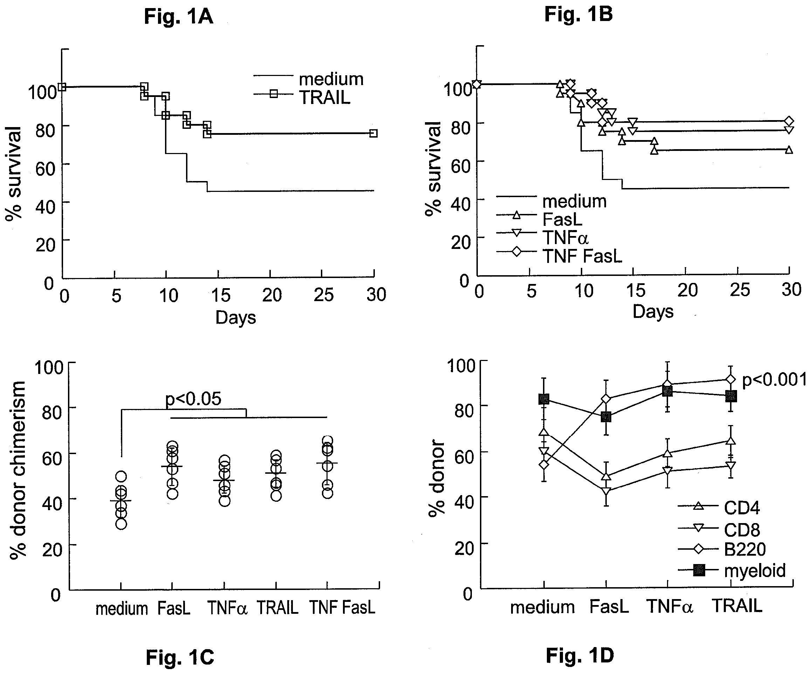

**Figure 1A** is a graph showing survival of mice grafted with cells incubated in medium and preexposed to 500 ng/ml TRAIL (n=20). There was no additional mortality at times longer than the 4 weeks presented here. **Figure 1B** is a graph showing survival of recipients of cells preincubated with 250 ng/ml FasL chimeric protein, 20 ng/ml TNFα and their combination (n=20). **Figure 1C** is a graph showing levels of donor chimerism at 3 weeks post-transplantation in the corresponding experimental groups (n=6 in each group). **Figure 1D** is a graph showing lineage chimerism in mice grafted with cells preexposed to the death ligands representing GR-1⁺ myeloid cells, CD4⁺ and CD8⁺ T cells and B220⁺ B lymphocytes.

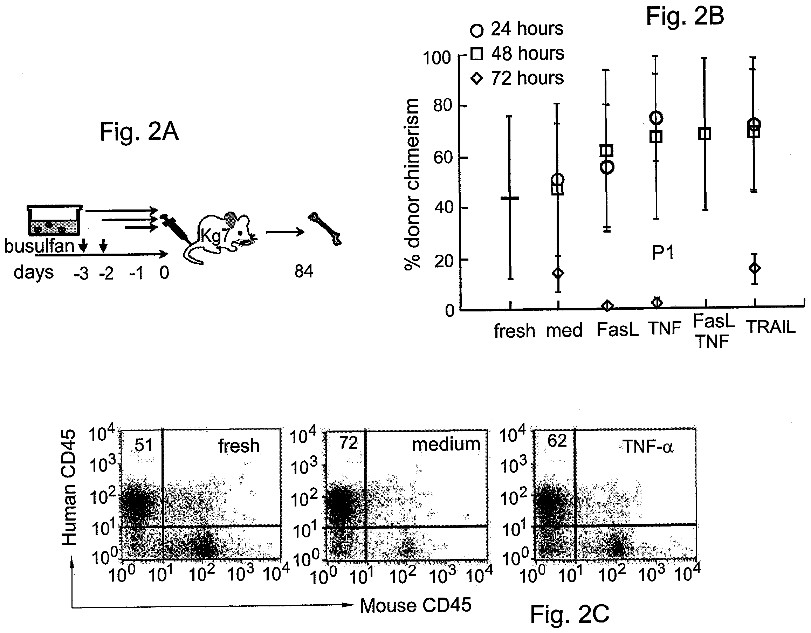

**Figure 2A** is a schematic diagram demonstrating the experimental outset. **Figure 2B** is a graph showing percentage of human chimerism determined by staining with human anti-CD45 monoclonal antibodies for fresh UCB (n=27) with pretransplant incubations of 24, 48 and 72 hours in medium (n=12, 10, 5, respectively) and with FasL (n=8, 8, 8, respectively), TNF-α (n=7, 7, 5, respectively), TRAIL (n=7, 8, 9, respectively). **Figure 2C** is a schematic representation showing representative measurements of human cell chimerism in murine bone marrow using species-specific CD45.

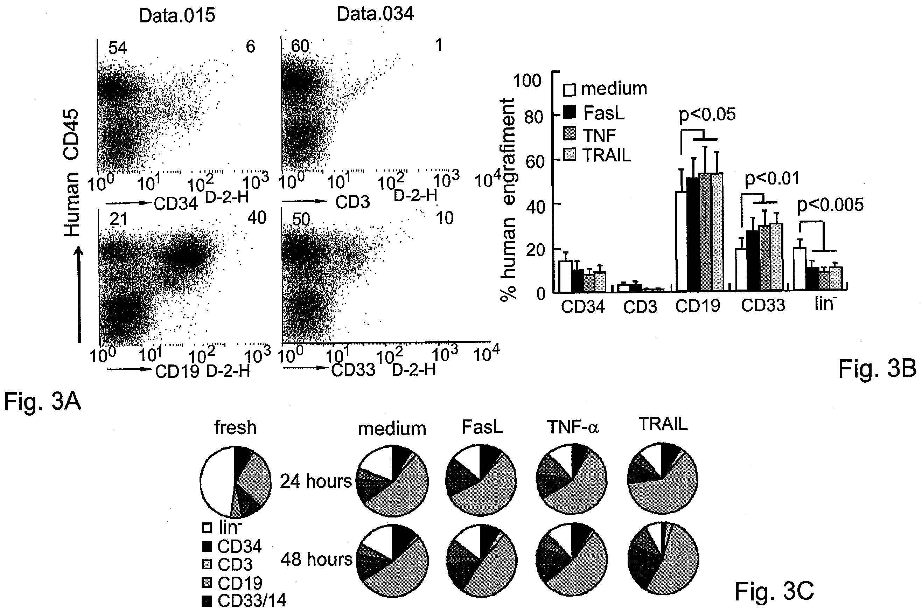

**Figure 3A** is a schematic representation of lineages developing from human UCB cells in the murine bone marrow at 12 weeks after transplantation. The transplanted cells were pre-incubated with 20ng/ml TNF-α prior to transplantation: CD34⁺ and lineage-negative (lin-) progenitors, CD3⁺ T cells, CD19⁺ B lymphocytes, CD33⁺ mycloid cells. **Figure 3B** is a graph showing the percentage of human cell engraftment in the bone marrow at 12 weeks after transplantation of cells preincubated for 48 hours with 50 ng/ml FasL, 20 ng/ml TNF and 1.5 µg/ml TRAIL for 48 hours (n=7-12 in each group). **Figure 3C** is a schematic representation showing qualitative engraftment after pre-exposure for 24-48 hours with the death ligands including CD14⁺ monocytes (n=4-7 in each group). Increased myelo-monocyte fractions are delineated.

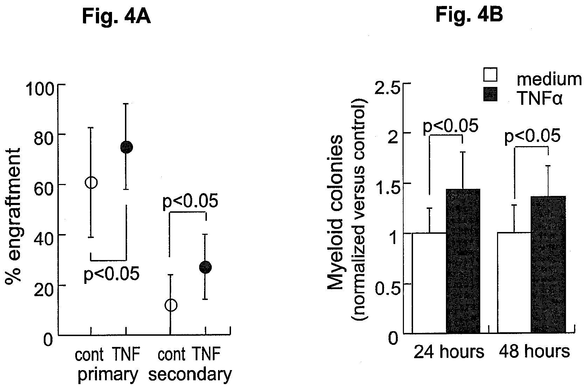

**Figure 4A** is a graph showing percent engraftment in primary and secondary recipients comparing control and TNFα treated UCB cells. **Figure 4B** is a graph showing the number of myeloid colonies in recipients of UCB cells treated with TNF or control (medium) for 24 hours or 48 hours.

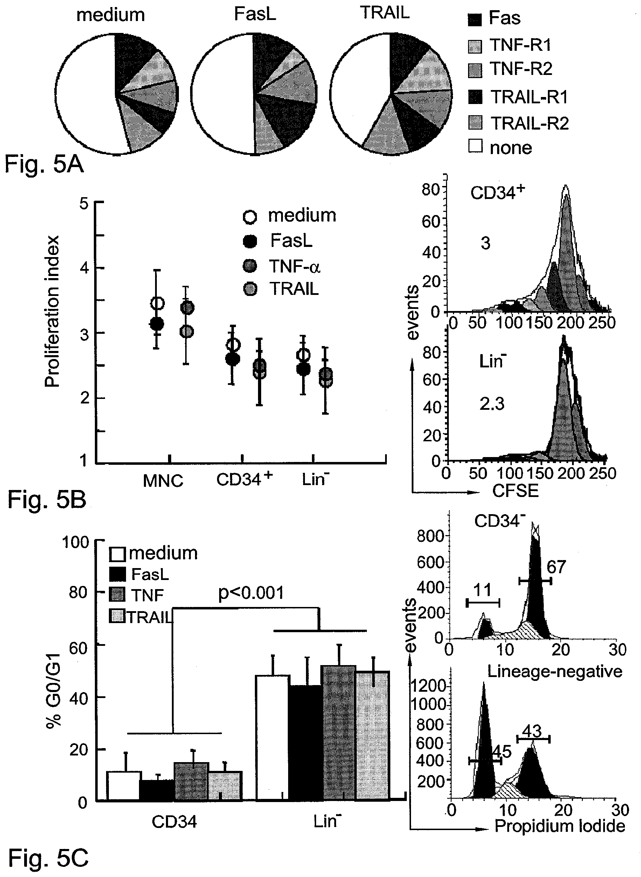

**Figure 5A** is a schematic representation of the fractional expression of Fas, TNF receptors and TRAIL receptors in UCB cells following incubation in medium and with 50 ng/ml FasL or 1.5 µg/ml TRAIL (n=5-9 in each group). **Figure 5B** is a graph showing the proliferation index of mononuclear (MNC) UCB cells, CD34⁺ and lineage-negative progenitors (lin) following 48 hours of incubation with FasL, TNF-α and TRAIL (n=4-7 in each group). Right panel presents demonstrative measurements of proliferation from CFSE dilution using the ModFit simulation software. **Figure 5C** is a graph showing the cell cycle phase in CD34⁺ and lin⁻progenitors following 48 hours of incubation with the ligands. Right panel is demonstrative for measurements of DNA contents using propidium iodide (n=4-7 in each group.

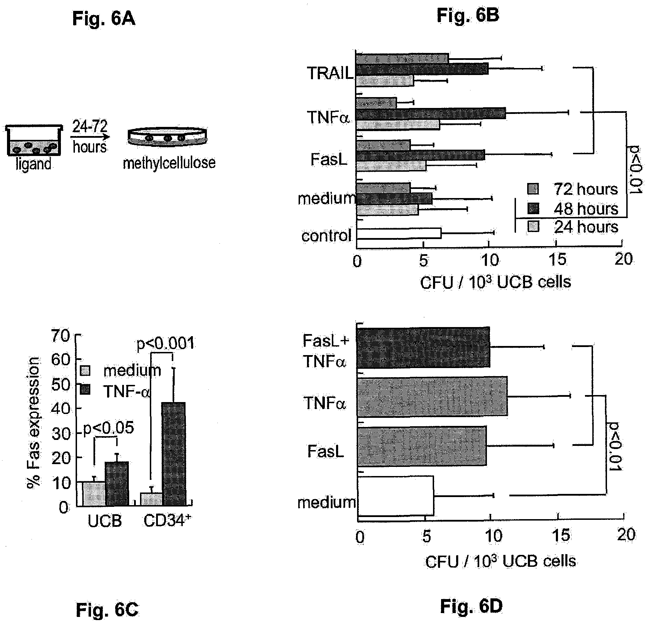

**Figure 6A** is a schematic diagram demonstrating the experimental outset. **Figure 6B** is a graph showing myeloid colony formation expressed per 10³ plated cells for fresh UCB (control) and after incubation in medium and with the death ligands for 24-72 hours (n=6-14 at each time point). **Figure 6C** is a graph showing percent Fas expression in CD34⁺ progenitors (n=9). **Figure 6D** is a graph showing myeloid progenitor activity in methylcellulose cultures (n=7-15).

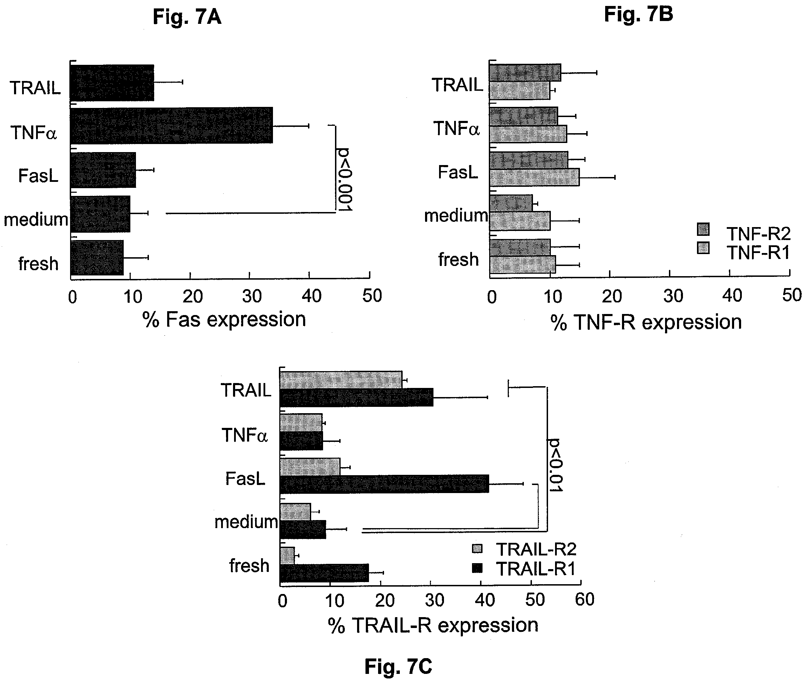

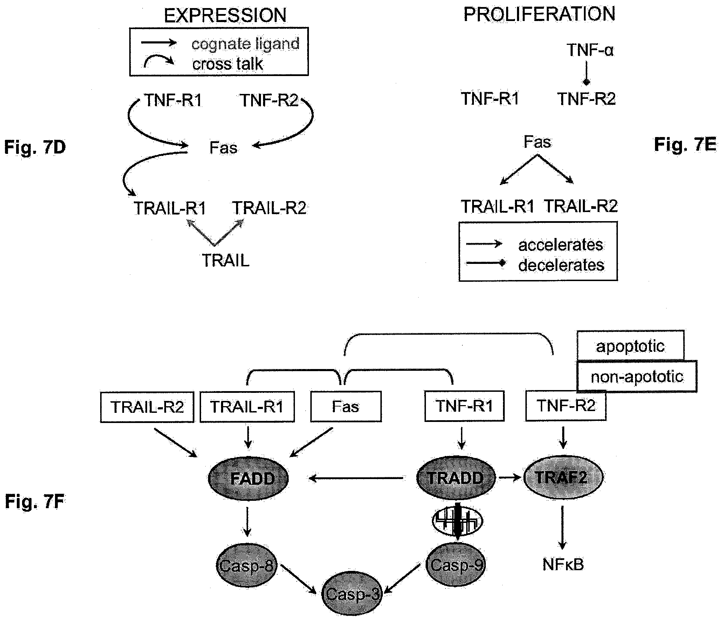

**Figure 7A** is a graph showing % Fas expression in fresh samples and following incubation with each of the ligands (n=29 fresh, n=11 incubated); **Figure 7B** is a graph showing % Fas expression in fresh samples and following incubation with each of the ligands TNF-R1 (n=7 fresh, n=5 incubated) and TNF-R2 (n=12 fresh and n=10 incubated); **Figure 7C** is a graph showing % Fas expression in fresh samples and following incubation with each of the ligands TRAIL-R1 (DR4, n=9) and TRAIL-R2 (DR5, n=8). Figure 7**D-F** Schematic representations of the consequences of receptor cross-talk and induction by cognate ligands: Figure 7**D** induced expression of receptors, Figure 7**E** attenuation of cell proliferation, Figure 7**F** apoptosis based on schematic representation of the submembranal signaling pathways.

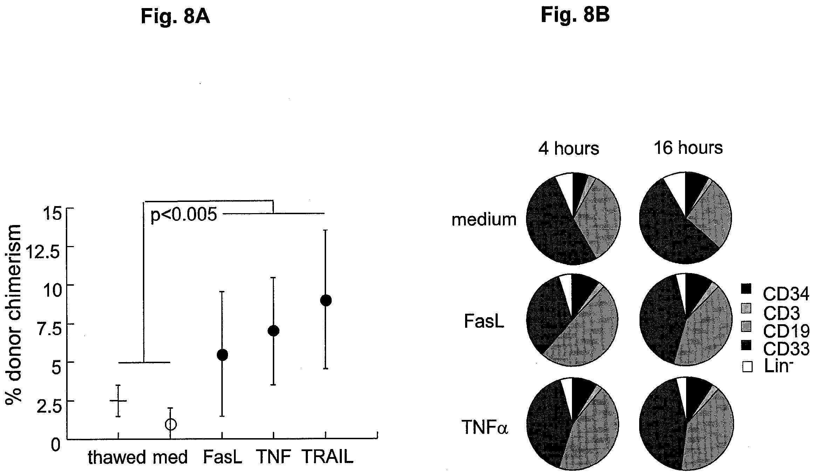

**Figure 8A** is a graph showing cumulative levels of human donor chimerism as determined from human anti-CD45 staining after transplantation of 11 mPB samples after incubation in medium and with the ligands (n=9-17 mice in each experimental group). **Figure 8B** is a schematic representation of fractional distribution of lineages in the host bone marrow at 12 weeks post-transplantation including: CD34⁺ and lineage-negative (lin⁻) progenitors, CD3⁺ T cells, CD19⁺ B lymphocytes and CD33⁺ myeloid cells (n=4-7 in each group).

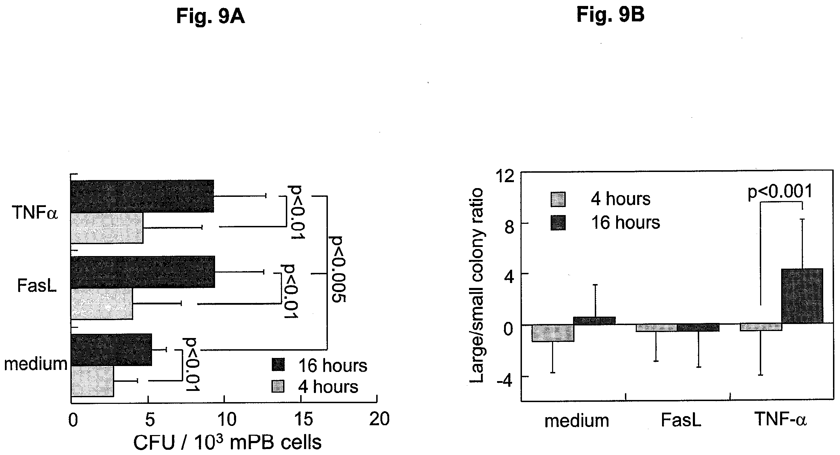

**Figure 9A** is a graph showing the number of CFU/10³ mPB cells. **Figure 9B** is a graph showing partition of large (>50 cells) and small (30-50 cells) myeloid colonies following incubation for 4 and 16 hours with the ligands (n=5-11).

### DETAILED DESCRIPTION OF THE INVENTION

The present invention relates to the employment of TNF family receptor signaling for inducing stem and progenitor cells activation. Because the apoptotic machinery is well conserved in all cells, including the most primitive stem and progenitor cells, this pathway is used for trophic signaling. According to the present invention, hematopoietic cell grafts are exposed to various combinations of ligands of the TNF family receptors prior to transplantation in order to induce their subsequent activity *in vivo.*

The present invention is based on the novel finding that pretransplant activation of the TNF family receptors on stem and progenitor cells (SPC), in particular on hematopoietic stem and progenitor cells (hSPC), improves the outcome of the transplant by fostering hematopoietic progenitor engraftment and reconstitution. Although each individual receptor is expressed in relatively small fractions of hematopoietic stem and progenitor cells, they are found in approximately 50% of fresh umbilical cord blood (UCB) progenitors. Therefore, joint activation has the capacity to affect the activity of significant fractions of progenitors. The results demonstrate that TNF family receptor activation is sufficient for induction of hematopoietic progenitor activity *in vivo.*

Moreover, the TNF family receptor activation is performed with TNF family ligands for a relatively short period of time in the absence of additional cytokines and growth factors. This approach is feasible for conditions of pretransplant preparation of grafts, which normally does not include supporting and stimulating factors.

In addition, the present invention differs from apoptosis-mediated mechanisms of graft preparation that use progenitor resistance to apoptosis for elimination of apoptosis-sensitive subsets responsible for GvHD and exclusion of residual malignant cells from autologous grafts. The time of exposure, the concentrations of the ligands and the nature of activated receptors to attain trophic pretransplant activation of progenitors are different from functional negative selection of progenitors based on their insensitivity to apoptosis (WO 2007/138597).

Umbilical cord blood is a rich source of hematopoietic stem and progenitor cells, the use of which increases exponentially in treatment of malignant and non-malignant disorders by transplantation. The main limitations of UCB as a source of donor cells are the small numbers of progenitors below the threshold required in transplants of adult recipients, and slow engraftment that extends the dangerous period of aplasia and host exposure to infections. Proposed solutions of these limitations include co-transplantation of several cord units and *ex vivo* expansion of progenitors. It has been previously demonstrated by the inventors that functional selection of UCB progenitors resistant to apoptosis increases their effective numbers as compared to isolation based on phenotypic markers (CD34, CD133). Furthermore, elimination of differentiated cells that become sensitive to apoptosis improves the yield of *ex vivo* expansion of UCB progenitors by awarding an advantage to uncommitted apoptosis-insensitive precursors.

The present invention provides an additional approach to shorten the time of engraftment by pretransplant activation of the progenitors, which is sustained and leads to accelerated quantitative and qualitative hematopoietic reconstitution.

Another prevalent source of hematopoietic progenitors is mobilized peripheral blood (mPB), based on collection of mononuclear cells by apheresis after stimulation of the bone marrow with mobilizing agents. The inventors have previously demonstrated that functional negative selection of T and B lymphocytes from mPB grafts reduces significantly the severity of GvHD while sustaining GvT activity [38]. This procedure improves significantly the efficiency of engraftment by retaining the graft facilitating effect of T cells with reduced GvHD capacity.

The present invention offers an additional approach to enhance quantitative and qualitative mPB cell engraftment by pretransplant activation of progenitors.

It has been previously suggested that progenitors are protected from apoptosis due to low levels of expression of receptors of the TNF family, which transduce apoptotic and suppressive signals [**8-10**]. Supporting evidence is the apparent protection of hematopoietic cells by blocking antibodies and soluble FasL [**14-16**], which exerts an anti-apoptotic effect by preventing Fas trimmerization [**2**]. *In vitro* experiments have demonstrated detrimental effects of TNF-α on the function of cytokine-activated hematopoietic cells [**17,18**] through induction of apoptosis **[19,20**], which is mediated both by direct interaction with its cognate receptors [**18,21,22**] and indirect upregulation of the Fas receptor [**8-10**]. In all these studies cells have been cultured for variable periods of time with various cytokines and chemokines *ex vivo*, conditions that are associated with dramatic phenotypic and functional changes [**23**], including induced Fas expression by cross-talk with TNF-α receptors [**12,16,18**]**.** Changes in cell function have been attributed to detrimental effects of the TNF family receptors, causing impaired homing, induction of cell cycling and loss of engraftment potential [**24-27**]. These data suggest that neutralization of the Fas and TNF receptors might be beneficial to hematopoietic cells function in particular in the transplant setting, due to involvement of this pathway in graft versus host disease.

On the other hand, acute upregulation of several TNF family receptors is observed in donor cells soon after homing to and seeding in the recipient bone marrow. The most primitive hematopoietic stem and progenitor cells ubiquitously upregulate the Fas [**28**], TNF [**29**], and tumor necrosis factor-related apoptosis-inducing ligand (TRAIL) receptors [**30**] under stress conditions. The physiological significance of these receptors has been emphasized by the defective engraftment and long-term reconstituting potential of progenitors deficient in Fas [**31**] and TNF receptors [**29**].

Cell activity is tightly regulated by mechanisms of activation and death, the major mechanism of irreversible negative regulation. The apoptotic machinery that controls cell viability is a well-conserved pathway expressed in all cells. however stem and progenitor cells are largely resistant to receptor-mediated apoptosis. It has been demonstrated that murine hematopoietic progenitors that upregulate the expression of the TNF family receptors after transplantation are largely insensitive to apoptotic signaling [**28, 29**]. These data have been extended to human progenitors derived from umbilical cord blood (UCB) and mobilized peripheral blood (mPB), which are resistant to apoptosis mediated by Fas (under consideration), TNF-R1 [**32**] and TRAIL-R1 [**30**].

In contrast with the art, the present disclosure concerns exposure of hematopoietic stem and progenitor cells to ligands of the TNF superfamily prior to transplantation in order to improve the outcome of the transplant by activating at least part of the transplanted cells. Without wishing to be limited by theory, the effect involves trophic stimuli mediated by the TNF superfamily receptors. Activation of each one of the receptors for Fas-ligand, TNF-a, and TRAIL or their combinations enhances quantitative and qualitative hematopoietic reconstitution. Pretransplant activation of the TNF superfamily receptors *ex vivo* has the capacity to modulate the subsequent behavior of progenitor cells *in vivo*, enhancing early hematopoietic reconstitution and fostering particular lineages according to pre-transplant duration and type of receptor activated. The nature, concentration of ligands, , combinations, duration of exposure are variably set according to the source of the graft, antigen disparity, size of donor inoculum (number of progenitors), condition of the recipients and anticipated morbidity in a particular setting. Receptors might be upregulated prior to or concomitant with their activation by application of the ligands.

Therefore, in a first of its aspects, there is disclosed a method for enhancing the engraftment of stem and progenitor cells (SPC) in a recipient in need of SPC transplantation, comprising:
a. contacting *ex vivo* a biological sample obtained from a donor, said biological sample comprising a population of SPC, with at least one member of the TNF super family or any fragment or derivative thereof; and
b. after said contacting step (a), transplanting said SPC into said recipient; wherein the transplanted SPC exhibit enhanced engraftment.

In another aspect, there is disclosed a population of cells for use in a method of transplantation, wherein said cells are stem and progenitor cells (SPC) with enhanced engraftment characteristics wherein said population of SPC with enhanced engraftment characteristics is obtained by contacting ex vivo a biological sample obtained from a donor, said biological sample comprising a population of SPC, with at least one member of the TNF super family or any fragment or derivative thereof.

In a specific embodiment said SPC are hematopoietic stem and progenitor cells (HSPC).

As used herein the phrase ***"enhancing the engraftment of stem and progenitor cells (SPC)"*** relates to an improvement in efficiency, quality or rapidity of cell transplantation, or accelerated quantitative and qualitative hematopoictic reconstitution which may result from the *in vitro* exposure and activation of the cells by a member of the TNF super family. For example, enhanced engraftment comprises increasing myeloid, lymphoid, thrombocytic or erythroid reconstitution or activity. Methods for assessing cell engraftment include, for example, cell migration and other *in vitro* techniques, as well as histological, immunological and/or radiological assessment of biological samples (e.g. a blood sample) as described in detail below.

As used herein the term ***"ex vivo"*** refers to a process in which cells that were removed from a living organism (preferably a human) in the form of a biological sample, are kept and optionally propagated outside the organism (e.g. in a test flask).

As used herein the phrase ***"population of cells"*** refers to a homogeneous or a heterogeneous isolated population of cells. ***"Population of SPC or HSPC"*** refers to a homogeneous or a heterogeneous isolated population of stem and progenitor cells or hematopoietic stem and progenitor cells, respectively.

As used herein the term ***"stem and progenitor cells"*** refers to the earliest renewable cell population responsible for generating cell mass in a tissue or body and the very early progenitor cells, which are somewhat more differentiated, yet are not committed and can readily revert to become a part of the earliest renewable cell population.

As used herein the term ***"hematopoietic stem and progenitor cells"*** refers to stem and progenitor blood cells that are responsible for generating all blood cell lineages.

As disclosed herein , the stem cells can be identified by stem cell markers such as CD34+, CD34+/CD38-, CD133+, CD34+/Lin- or other stem cell markers known in the art.

The source of the SPC may be umbilical cord blood (UCB), mobilized peripheral blood (mPB), or bone marrow. Namely, in a specific embodiment the biological sample comprising a population of SPC or HSPC is selected from the group consisting of umbilical cord blood (UCB), mobilized peripheral blood (mPB). or bone marrow.

Methods of preparation of cells for transplantation are well known in the art. Tissue (e.g. umbilical cord blood and bone marrow aspirate) is removed using a sterile procedure. Cells prepared for transplantation can be maintained in a physiological solution, or cultured in suspension or on a fixed substrate. Suitable culture media capable of supporting cells include HEM, DMEM, RPMI, F-12, and the like. If required, the medium can contain supplements required for cellular metabolism such as glutamine and other amino acids, vitamins, minerals, transferrin and the like. The medium may also contain antibiotics to prevent contamination with yeast, bacteria and fungi, such as penicillin, streptomycin, gentamicin and the like. In the culture, conditions should be close to physiological conditions (preferably, a pH of about 6 to about 8, and a temperature of about 30°C to about 40°C, preferably about 37°C).

Cells may be obtained from umbilical cord blood (UCB) according to any method known in the art, for example, cells are obtained from UCB after normal full-term delivery (after receipt of informed consent). Samples can be collected and frozen for example according to Rubinstein et al (PNAS USA 1995; 92 (22):101 19-10122) within 24 hours postpartum. Prior to use, the cells can be thawed in Dextran buffer containing 2.5% human serum albumin, layered on a Ficoll-Hypaque gradient and centrifuged at 800xg for 30 minutes. The mononuclear cells in the interface layer may be collected and washed three times in phosphate-beffered saline (PBS) comtaining 0.5% HSA.

The biological sample may be freshly harvested, preserved, or cryopreserved. Fresh or cultured cell preparations can be cryopreserved until they are needed, by any method known in the art. The cells can be suspended in an isotonic solution, preferably a cell culture medium, containing a particular cryopreservant. Such cryopreservants include dimethyl sulfoxide (DMSO), glycerol and the like. Further methods for preparation and storage of cells for transplantation are known in the art, and disclosed in detail in, for example, the Handbook of Transplantation (Kipshidze and Serruys, eds. London, UK, 2004)

Said enhanced engraftment comprises increasing myeloid, lymphoid, thrombocytic or erythroid reconstitution or activity.

The ***"tumor necrosis factor (TNF) superfamily"*** currently consists of 19 ligands and 29 receptors in humans, with three additional TNF superfamily receptors having been identified in mice. Most TNF ligands are type II transmembrane proteins whose extracellular domains can be cleaved by specific metalloproteinases to generate soluble cytokines. Receptors for TNF superfamily ligands are oligomeric, type I or type III transmembrane proteins. Members of the TNF superfamily include 4-1BB/TNFSF9, APR1L/TNFSF13, BAFF/BLyS/TNFSF13B, CD27 Ligand/TNFSF7, CD30 Ligand/TNFSF8, CD40 Ligand/TNFSF5, EDA/Ectodysplasin, EDA-A1/Ectodysplasin A1, EDA-A2/Ectodysplasin A2, Fas Ligand/TNFSF6, GITR Ligand/TNFSF18, HGHT/TNFSF14, Lymphotoxin. Lymphotoxin beta/TNFSF3. Lymphotoxin-alpha/TNF-beta, OX40 Ligand/TNFSF4, TL1A/TNFSF15, TNF-alpha, TRAIL/TNFSF10, TRANCE/TNFSF11/RANK L, TWEAK/TNFSF12.

All receptors and ligands in this family display significant structural homology (-85%). However distinct active terminals mediate selective binding of the ligands to cognate receptors without cross activation within the family. TNF has two cognate receptors, and TRAIL has four membrane-associated receptors and one soluble receptor, of which three (including the soluble) are considered to be decoy receptors that bind the ligand without signal transduction. Within the TNF family Fas is considered to be the common executioner of apoptosis, Fas presents several distinct characteristics as compared to other members of the family **[1, 2**]**.** Activation of Fas requires receptor trimerization therefore the active isoform of FasL is membrane bound under physiological conditions and needs conjugation to generate oligomers for pharmacological uses. The soluble isoform of FasL has anti-apoptotic activity, as ligation of Fas without trimerization blocks apoptotic signal transduction.

As disclosed herein, the at least one member of the TNF super family is selected from the group consisting of Fas ligand (FasL), tumor necrosis factor α (TNF-α), and tumor necrosis factor - related apoptosis inducing ligand (TRAIL).

Said biological sample is UCB and said contacting of step (a) is for between about 18 hours and about 48 hours.

As also disclosed herein, said biological sample may be bone marrow and said contacting of step (a) is for between about 12 hours and about 32 hours.

As also disclosed herein, said biological sample may be mPB and said contacting of step (a) is for between about 3 hours and about 18 hours.

As disclosed herein, the SPC arc incubated with TNF-α at a concentration of between about 10ng/ml and about 20ng/ml, and/or FasL at a concentration of between about 10ng/ml and about 50ng/ml, and/or TRAIL at a concentration of between about 500ng/ml and about 1500ng/ml.

As disclosed herein, the SPC are incubated with the at least one member of the TNF super family or any fragment or derivative thereof in the absence of any additional cytokine, chemokine or growth factor.

As used herein the term ***"fragment or derivative"*** of a member of the TNF super family relates to any portion of the protein which retains the activity of the complete protein, with respect to increasing engraftment as defined above.

In a specific embodiment, said at least one member of the TNF super family is conjugated to a surface, e.g. a bead. The conjugation may be via a linker.

In a specific embodiment, said at least one member of the TNF super family is a combination of TNFα and FasL.

As demonstrated in the Examples below, pretransplant exposure of murine bone marrow-derived progenitors to ligands of the TNF family improved recipient survival under limiting dilution conditions. In addition, activation of the progenitors with each one of the ligands improved substantially quantitative and qualitative hematopoietic reconstitution. These data document the capacity of the TNF family receptors (via exposure to ligands of the TNF family) to activate murine progenitors and enhance early engraftment to rescue irradiated mice.

Pretransplant exposure to TNF family ligands prior to transplantation improves quantitative engraftment of human UCB and mPB cells. The SCID reconstituting cell (SRC) is considered to be the most reliable surrogate assay for human cells, which is related to the human reconstituting cell (HRC). However wide variability between human cell samples and biological variability of engraftment in individual mice are significant drawbacks of this assay. In addition, the human cells develop under conditions of partial mouse-human compatibility of cytokines and growth factors. Comparative analysis of impact of death ligands on engraftment was therefore performed using a relatively large number of human samples (65 UCB units), each one grafted into 3-7 recipients. As shown in the Examples below pretransplant activation of the TNF family receptors resulted in superior quantitative engraftment of fresh UCB cells and cryopreserved mPB samples. Overall, pretransplant exposure of human cells to ligands of the TNF superfamily improves quantitative human cell engraftment in SCID xenochimeras.

Pretransplant exposure to TNF family ligands modulates qualitative engraftment of human UCB and mPB cells. B lymphocytes and myeloid cells are the first human lineages that develop from human progenitors in SCID mice. As shown in the Examples, the fractions of B lymphocytes were enhanced and superior myeloid engraftment was demonstrated in recipients of UCB cells incubated with the TNF family ligands. Thus, improved quantitative engraftment is a result of early engraftment and enhanced function of the progenitors. In addition, the time of pretransplant activation of the TNF family receptors modulates the quality of hematopoietic progenitor function.

Activation of hematopoietic progenitors through ligation of the TNF receptors evolves as a common pathway in murine and human cells. Activation persists *in vivo*, as emphasized by superior quantitative and qualitative engraftment, as demonstrated in the Examples. This evidence points to enhanced engraftment and early progenitor activity mediated by activation of the TNF superfamily receptors.

Umbilical cord blood is a rich source of hematopoietic progenitors as compared to bone marrow and mobilized peripheral blood. The high frequency of SRC was evident in the superior levels of human chimerism in mice grafted with UCB cells as compared to 10⁷ mPB cells. The main limitations of umbilical cord blood transplants are the low numbers of progenitors and the slow tempo of engraftment. UCB progenitors are slow to initiate activity because their naïve and primitive nature, and absence of prior experience in interaction with the bone marrow stroma. Durable multilineage human hematopoietic reconstitution is mediated only by progenitors engrafted in the bone marrow. The present invention has the capacity to affect both limitations: earlier engraftment and improved quantitative reconstitution.

The present invention is suitable for use in any type of transplant, i.e. from different sources of donor cells and variable conditions of the recipients. The potential complications can be often estimated prior to transplantation, making pretransplant modulation of the graft clinically relevant. The time to engraftment is one of the significant parameters of the transplant, with significant impact on susceptibility to infection, the requirement of transfusions during the period of aplasia, bleeding and veno-occlusive events that cannot be treated prior to platelet recovery.

It is contemplated that post-transplant administration of G-CSF is more effective in fostering donor cell engraftment after pre-transplant activation of the progenitors through TNF family receptor ligation.

Mobilized peripheral blood is an accessible source of cells with wide use in oncological transplants, which is limited by potentially-lethal GvHD mediated by mature donor T cells. In addition to reduction of GvHD severity by functional depletion of apoptosis-sensitive T cells [**36, 38**], the proposed approach is likely to impact on graft versus tumor reactivity by augmenting engraftment. The period of immunesuppression during transplantation is dangerous due to unrestricted growth of tumor cells.

The true nature of progenitor activation is emphasized by the secondary transplant experiments. One of the questions imposed by progenitor activation is whether it causes extinction through differentiation of progenitors endowed with short-term and long-term hematopoietic reconstituting capacity. Increased B lymphocyte and myeloid progeny were accompanied by reciprocal decrease in lineage-negative cells in the bone marrow of primary SCID recipients. However, along superior quantitative reconstitution in primary recipients, the reconstitution of sequential secondary recipients was improved as well. Without wishing to be bound by theory, these results indicate that the TNF family receptors improve engraftment by progenitor activation rather than extinction.

As shown previously, progenitors are induced to engage to clonogenic activity through synergism with stimulating factors such as granulocyte macrophage colony stimulating factor (GM-CSF) and stem cell factor (SCF) **[29,30**]. These signals are mediated in murine cells by Fas, TNF-R1 and TRAIL-R2 (the only murine TRAIL receptor) **[29-311**]. Consistently, trophic signals of ligands continuously present in cultures synergize with growth factors to stimulate the activity of human UCB and mPB samples **[36, 38**]. These signals are mediated primarily by TNF-R1 and TRAIL-R1, with lesser positive influence of Fas. At the same time, TNF family ligands act as negative regulators in the terminally differentiated progeny, decreasing the size of colonies with significant variance between the individual receptors **[29-31**]. One significant limitation of these surrogate assays of human cells is the negative imact of apoptotic cells on development of colonies, which is exaggerated by induction of apoptosis in sensitive cells by the death ligands [**29,32**]. Therefore, the true trophic effect of the ligands in culture is an underestimate mediated by an intrinsic factor because the differentiated progeny becomes sensitive to apoptosis even if cultures are initiated with purified progenitors.

The mechanism of stem and progenitor cell activation in accordance with the present invention is exemplified by the increased numbers of myeloid colonies of cells harvested from the bone marrow of primary recipients. Even at 12 weeks post-transplantation the clonogenic activity was increased in recipients of cells pretreated with TNF-α. This *in vitro* assay is consistent with and validates enhanced myeloid activity *in vivo.* Therefore, pretransplant progenitor activation through TNF superfamily receptors has sustained posttransplant consequences.

*In vitro* clonogenic assays were performed to validate the consequences of TNF family receptor ligation observed *in vivo.* Clonogenic activity in semisolid methylcellulose cultures reflects the activity of a more committed subset of progenitors as compared to the SCID reconstituting cell, which are stimulated by growth factors such as SCF, IL-3 and GM-CSF. The clonogenic assays largely confirm enhanced activity following short-term incubation with the TNF family ligands of UCB and mPB cells. These data demonstrate stimulation of committed hematopoietic progenitors by the TNF family receptors. Importantly, the clonogenic assays performed here are underestimates of the true inductive effect of the TNF family receptors because substantial numbers of dead cells were included in the cultures. The presence of dead cells substantially inhibits clonogenic activity in semoisolid cultures [**32**]. These assays demonstrate direct trophic effects and are distinct from increased progenitor frequency within the viable population achieved by elimination of dead cells [**33,34**].

Differentiated hematopoietic cells acquire sensitivity to apoptotic signaling along the process of maturation, with the TNF family receptors becoming the major homeostatic mechanism of negative regulation. This was evident from decreased colony size in the presence of death ligands in the cultures [**29-32**]. However, as shown in the examples below, the inductive activity of TNF-α. was evident in the large colony size following preactivation of mPB progenitors. The ligands were not present in culture and therefore did not impose negative regulation on the differentiated progeny.

As shown in the examples below, some of the characteristics of progenitors were revealed by analysis at the end of the short-term incubation period with TNF family ligands. Activation of the receptors did not affect the fractions of mitotically-quiescent cells. These cell contain the SRC subset [**24-26**]. Furthermore, activation of the receptors did not induce proliferation of CD34⁺ and lineage-negative UCB progenitors. These data demonstrate that the TNF family receptors activate progenitor activity without amplification of their numbers or induction of proliferation.

As opposed to the traditional dogma [**8-10, 17-22, 27**], it is emphasized that activation of TNF family receptors does not have detrimental consequences on hematopoietic progenitor viability and function. Prior studies reporting detrimental effect of the TNF family receptors on hematopoietic cell function have stimulated the progenitors *in vitro,* by supplementation of growth factors and supporting chemokines, as well as their withdrawal [**12,13,16-21,27,35**]. As shown in the present invention, experiments were designed to simulate the clinical setting, where progenitors are not stimulated and supplemented with supporting chemokines prior to transplantation. In contrast to the previous art, the inventors of the present invention found significant trophic activity of TNF receptor activation in progenitors. The data indicates that receptor-mediated signaling induces trophic pathways, which transduce persistent activation and dominate subsequent progenitor function.

As shown below, although each receptor is expressed in small fractions of fresh UCB cells, approximately 50% of the progenitor cells are responsive to signaling through one of the receptors. The trophic receptors in human cells are TNF-R1 with unknown activity of TNF-R2 [**32**], and TRAIL-R1 with antagonizing activity of TRAIL-R2 [**30**].

The present invention takes into consideration the following elements: a) the target receptor of activation, b) concentrations of the ligands, c) duration of receptor ligation to achieve progenitor activation, d) modulation of receptor expression, and e) combinations of the applied ligands.

The non-limiting Examples below show the activation of Fas, TNF and TRAIL receptors. Each one of these receptors displays trophic activity in hematopoietic progenitors. In murine cells there are similar consequences of activation of the three receptors at optimal concentrations of the ligands. In variance, in human cells of both UCB and mPB origin, TNF-R1 and TRAIL-R1 are more potent in induction of activity than Fas. The effective concentrations of the ligands for human progenitor activation are in the range of: FasL 10-50 ng/ml, TNF-α 10-20 ng/ml, and TRAIL 500-1500 ng/ml (at suspension concentrations of 1-5x10⁷ cells/ml).

The duration of exposure to the ligands differs. UCB cells are incubated for periods of 18-48 hours, bone marrow cells for 12-32 hours, mPB for 3-18 hours. The periods of incubation for trophic signaling are different and generally longer than the time of exposure for functional negative selection of progenitors. The period of pretransplant incubation of human cells is limited by the dramatic phenotypic changes these cells undergo in culture and loss of engraftment potential after three days of *ex vivo* culture. As shown in the examples below, loss of engraftment potential of UCB progenitors after *ex vivo* culture for 72 hours is independent of apoptotic signaling mediated by the TNF family receptors. Therefore, the general aim is to shorten the period of culture to the minimum. As detailed above, the time of incubation also determines preferential induction of lymphoid and myeloid differentiation.

The use of combinations of TNF family ligands offers the opportunity to tailor and individualize the composition of the pretransplant incubation conditions. One such combination is presented for FasL and TNF-α, showing similar trophic activity as each ligand alone.

Resistance of progenitors to apoptosis offers the opportunity to modulate the pattern of responsiveness to various members of the TNF family ligands by modulation of receptor expression. For example, the Fas and TNF receptors may be upregulated by interferon-γ, prior to or concomitant with application of the cognate ligands. Modulation of the patterns of receptor expression has the capacity to attenuate the responses of progenitors. The use of several ligands allows a certain degree of flexibility in pretreatment of cells from various sources under different transplant settings, as an approach to promote engraftment. Notably, there is no cross-reactivity between TNF family receptor sand ligands, and each molecule is restricted to its cognate receptors.

Besides direct activation by the cognate ligands, inductive cross-talk increases the propensity of various receptors. As shown in the examples below the well-characterized inductive effect of TNF-α on Fas expression **[8, 12**] is mediated by both TNF receptors. In addition, additional inductive interactions were revealed: Fas induces expression of TRAIL-R1 and TRAIL induces expression of both TRAIL-R1 and TRAIL-R2. The functional consequences of receptor cross-talk include induction of proliferation of progenitors expressing TRAIL-R1 and TRAIL-R2 in response to Fas cross-linking and additive apoptosis of cells co-expressing Fas and TNF-R2. However, joint expression of Fas and TNF-R1 or TRAIL-R1 does not increase the susceptibility to apoptosis. In fact, TNFα-induced expression of Fas in -40% of CD34⁺ UCB progenitors and cross-linking by FasL does not impair stimulation of myeloid progenitors in semisolid cultures. Altogether, the data presented below emphasizes a significant cross-talk between TNF family receptors, which is dissociated from apoptotic signaling in hematopoietic progenitors.

The differences between influences of the different ligands offer the possibility of using mixtures to attain variable outcomes for particular subsets of hematopoietic cells from various sources. However, other considerations may impact on the determined optimal combination of ligands, such as the degree of donor-recipients HLA mismatch, ABO incompatibility, the absolute numbers of cells and progenitors available for transplantation (at an approximate threshold of 5x10⁶ CD34⁺ cells/kg), active malignant disease (blast crisis) versus minimal residual disease (1:10,000 malignant cells) and complete remission, preexisting infections that might be reactivated during the post-transplant period of neutropenia and lymphopenia (for example CMV, EBV, Varicella, Herpes), ongoing chronic GvHD (for example after previous unsuccessful transplant), the threat of acute GvHD (for example haploidentical transplants), host resistance to engraftment (for example Fanconi anemia), as few examples of transplant-related considerations. Similar criteria will be applied to autoimmune disorders, which may be of predominant aberrant myeloid (for example Rheumatoid Arthritis) or lymphoid cell etiology (for example Type 1 diabetes, Multiple sclerosis, Systemic lupus erythematosus, Inflammatory bowel disease).

### EXAMPLES

### Example 1: Ex vivo exposure of murine bone marrow to death ligands enhances progenitor function

This example demonstrates the impact of TNF family receptor activation, prior to transplantation, on murine bone marrow-derived progenitor engraftment. Recipient mice conditioned with 850 rad TBI were grafted with 1.5x10⁵ lineage-negative syngeneic (CD45.1→CD45.2) progenitors after incubation for 18 hours in medium and in the presence of death ligands. Because bulk cell incubations are contaminated by the contents of dead cells that reduce the clonogenic activity of progenitors [**29, 311**], isolated lineage-negative progenitors were exposed to the death ligands prior to limiting dilution transplants. Survival of -50% of the mice is conferred by transplantation of borderline numbers of cells, i.e. 1.5x10⁵ syngeneic bone marrow-derived lin⁻ progenitors. Transplantation of 1.5x10⁵ lin- progenitors prestimulated with TRAIL (**Figure 1A**), FasL and TNF-α (**Figure 1B**) for 18 hours resulted in substantially improved superior survival. In addition, joint exposure to TNF-α to induce Fas receptor expression, and receptor cross-linking with FasL resulted in equal superior survival in limiting dilution transplants (**Figure 1B**). These data emphasize that short-term pretransplant stimulation of murine bone marrow cells with death ligands is physiologically significant to the extent of improving survival under limiting dilution conditions.

To validate the inductive effect of receptor activation on engraftment among several possible mechanisms, donor cell development was monitored in the mixed chimeras. Recipients of cells preexposed to either one of the death ligands and the combination of TNF-α and FasL showed elevated levels of donor chimerism at three weeks after transplantation (**Figure 1C**). To refine the inductive effect of death receptor activation on the progeny in mixed chimeras, the progeny of bone marrow precursors was immunophenotyped. Myeloid engraftment was stable at 3 weeks post-transplantation, which is most probably caused by saturation values under these transplant conditions (**Figure 1D**). In variance, lymphocytic compartment presented a modest decline in both CD4⁺ and CD8⁺ T cells and a significant increase in B lymphocytes. Since B cells are the first lymphocytic lineage to develop following transplantation, these data clearly indicate an inductive effect of receptor activation n progenitor function.

### Example 2

### Pretransplant ex vivo exposure of UCB cells to death ligands enhances engraftment

The significant limitations of UCB cells are the low numbers of progenitors and the slow tempo of engraftment of these marrow-inexperienced cells. Fresh umbilical cord blood units were incubated for variable periods of time (1-3 days) in medium (without supplementation of chemokines) and with 50 ng/ml FasL, 20 ng/ml TNF-α and 1,500 ng/ml TRAIL. Equal numbers of initial cells were grafted into immunocompromized NOD.SCID mice conditioned with two doses of 25 µg/g busulfan. For comparison cells from the same UCB unit were grafted both as fresh or medium-incubated, and after exposure to the death ligands. Readouts were performed 12 weeks post-transplantation in the murine bone marrow (**Figure 2A**). Summary of transplant experiments from 9 cord units (each cord unit served for transplantation of 3-6 mice in 3 experimental groups) showed a trend of increased chimerism following pre-transplant exposure with FasL, TNF-α and TRAIL for 24-48 hours as compared to medium alone (Figure 2B). Demonstrative measurements of human chimerism in murine bone marrow are shown for preincubation with TNF-α. Similar results were obtained when UCB cells were preexposed to a truncated isoform of metalloproteinase cleavage site-deficient FasL conjugated to streptavidin [**28, 31, 37**]. Importantly, TNFα induces Fas receptor expression (vide infra), however joint incubation with FasL does not impair enhancemnt of human xenochimerism. Incubation of UB cells for periods exceeding 2 days is associated with marked decrease in engraftment efficacy irrespective of the presence of ligands. These data document enhanced quantitative UCB cell engraftment induced by pretransplant exposure to death ligands of the TNF superfamily.

### Example 3

### Pretransplant exposure of UCB cells to death ligands enhances qualitative myeloid reconstitution

The time to engraftment in the clinical setting is determined from stable presence of granulocytes in peripheral circulation (>500 cell/dl), associated with reduced susceptibility to infection. To evaluate the impact of the death ligands on UCB progenitors *in vivo*, the human hematopoietic lineages in the murine bone marrow were assessed. An example is shown for cell pretreated with TNFα showing dominant B lymphocyte progeny with minor T cell development in gates on human cells (**Figure 3A**). Mice were grafted according to the conditions shown in figure **2A****.** Pre-exposure of UCB cells to the death ligands before transplantation modulates the pattern of engraftment, with increased myelo-monocyte progeny (p<0.05) and increased B lymphocyte chimerism, which is the first human hematopoietic lineage to develop in NOD.SCID mice (p<0.01, **Figure 3B**). Induction of human progenitor activity was consistent with a reciprocal decline in numbers of lineage-negative human cells in the murine bone marrow (p<0.005). Notably, the most significant inductive effect was observed after 48 hours of incubation of UCB cells, and persisted during extended periods of engraftment of 12 weeks *in vivo* (**Figure 3C**). These data document enhanced early human myeloid and B lymphocyte progenitor activity in vivo following pretransplant induction by the death ligands. The SCID reconstituting cell (SRC) is considered to correlate best with the activity of short-term human reconstituting cells.

### Example 4

### Enhanced progenitor activity improves secondary reconstitution

Activation of SCID reconstituting cells might cause extinction of the human progenitors and impair durable engraftment. However, the absolute contents of human CD34⁺ cells in murine bone marrow at 12 weeks post-transplantation were increased by preexposure to TNF-α for 24 and 48 hours by 34±30% (non-significant) and 21% ± 24% (non-significant) as compared to control medium, respectively. Therefore, the human cells engrafted in primary recipients were further assessed in a functional assay of engraftment in secondary recipients. Primary recipients were grafted according to the conditions shown in figure 1A. In the first stage the impact of TNF-α on progenitors responsible for durable reconstitution was determined by transplantation of the marrow contents of primary recipients into secondary recipients. At 12 weeks post-transplantation half of the femoral cellular contents of primary recipients of UCB cells preincubated for 24 hours in medium and with TNFα were grafted into secondary busulfan-conditioned NOD.SCID mice. Half of the femoral contents of the primary recipients of cells preincubated with TNFα contained 3.2±1.4x10⁵ as compared to 5.3±3.1x10⁵ human CD34⁺ cells preincubated in medium (not significant) for 24 hours. Human xenochimerism was measured in the bone marrow after 12 weeks (3 UCB units in 6 mice in each group). Transplantation of half of femoral contents into secondary NOD.SCID recipients displayed increased levels of human chimerism following preexposure to TNF-α (p<0.05, **Figure 4A**), validating the absence of detrimental effects of the cytokine on human progenitors. In second stage the apparent increase in myeloid phenotypes in the bone marrow *in vivo* Was further assessed in methylcellulose cultures stimulated with human cytokines *in vitro.* Equal numbers of CD34⁺ human progenitors derived from the bone marrow of primary recipients were assessed at the experimental end point in semisolid methylcellulose assays stimulated with human SCF, IL-3 and GM-CSF. Colonies of recipients of UCB cells preincubated for 24 hours (n=6 from 3 UCB units) and 48 hours (n=7 from 3 UCB units) were normalized to the clonogenic activity measured in recipients of cells preincubated in medium (normalized). Increased myeloid clonogenic activity following exposure to TNF-α for 24 and 48 hours was demonstrated (**Figure 4B**). Increased progenitor numbers and enhanced myeloid repopulation point to effective self-renewal of UCB progenitors that is augmented by *ex vivo* exposure to TNF-α. (as a demonstrative ligand).

### Example 5

### Characterization of the influences of UCB cell preincubation

In next stage we characterized some of the characteristics of UCB cells during pre-transplant incubation and exposure to the TNF family ligands. The impact of the ligands on UCB cells depends on expression of the cognate receptors. All receptors are expressed at relatively low levels in UCB cells incubated for 48 hours in medium, with variable influences of FasL and TRAIL (**Figure 5A**). The influence of TNF-α is detailed below. Exposure to either one of the ligands does not induce proliferation, with slow cycling of CD34⁺ and lineage-negative progenitors ac compared to bulk lineage-positive UCB cells in liquid culture (**Figure 5B**). Likewise, the ligands do not affect the cycle phase, with approximately 50% of lineage-negative progenitors found in the GO/G1 phase, whereas only a minute fraction of CD34⁺ progenitors are mitotically quiescent (**Figure 5B**). This pattern is consistent with preserved and enhanced engraftment, since SRC (and human reconstituting cell) activity is confined to the subset of mitotically quiescent progenitors [**24-26**].

### Example 6

### Exposure of UCB cells to death ligands enhances myeloid progenitor activity in semisolid cultures

The impact of UCB cell preincubation with the TNF family ligands on myeloid progenitor activity was further determined in semisolid methylcellulose cultures stimulated with SCF, IL-3 and GM-CSF (**Figure 6A**). UCB cells were exposed to 50ng/ml FasL, 20ng/ml TNF-α and 1,500 ng/ml TRAIL for 1-3 days. To determine progenitor stimulation, equal numbers of total preincubated cells were plated in semisolid methylcellulose cultures irrespective of viability (namely, without elimination of dead cells). Cultures were stimulated with granulocyte-macrophage colony stimulating factor (GM-CSF), interleukin-3 (IL-3) and stem cell factor (SCF). Exposure of UCB cells to all ligands for 48 hours stimulated myeloid progenitor activity in semisolid cultures as compared to fresh cells and incubation in control medium (**Figure 6B**). For comparative analysis same UCB samples were plated as fresh or control and following exposure to the ligands. One exception was enhanced clonogcnic activity after 72 hours of incubation with TRAIL, despite significant reduction in SRC activity (**Figure 2B**). Exposure of UCB cells to TNF-α for 48 hours upregulates Fas expression in -50% of CD34⁺ UCB progenitors (**Figure 6C**). Because TNF-α is a potent inducer of Fas expression, we assessed the impact of this induction on progenitor activity *in vitro.* Joint exposure of UCB cells to 50 ng/ml FasL and 20 ng/ml TNF-α was also effective in myeloid progenitor activation, comparable to the impact of each of FasL and TNF-α alone (**Figure 6D**). These data demonstrate that TNF-induced Fas expression does not sensitize progenitors to apoptosis however their effect is redundant rather than additive or synergistic. These in vitro data are consistent with and explain the stimulatory effect of TNF family ligands on myeloid progenitor activity in vivo.

### Example 7

### Cross-talk of TNF family receptors

Although the TNF family receptors display redundant activity in clonogenic assays, their differential influences might require application of combinations of activating ligands to attain various tasks. In this example the presence of inductive cross-talk between the receptors, which might sensitize to apoptosis and trophic influences, was assessed. Dynamics of death receptor expression in liquid culture were examined. Mononuclear UCB cells were incubated for 72 hours with 50 ng/ml FasL, 20 ng/ml TNF-α and 1.5 µg/ml TRAIL, and expression of the receptors was determined by gating on CD34⁺ progenitors in fresh samples and following incubation with each of the ligands. As emphasized in prior studies [**8, 12**], TNF-α is the only ligand that induces expression of Fas in CD34⁺ UCB progenitors (**Figure 7A**). In variance from absent influence of the ligands on expression of the TNF receptors (**Figure 7B**), FasL induces significant expression of TRAIL-R1 in progenitors (**Figure 7C**). In addition, TRAIL induces its own receptors, which might be responsible for the sustained positive influences after extended incubation periods of 72 hours (**Figure 6B**). In summary, remarkable cross talk is observed between both TNF receptors and Fas (**Figure 7D**), while only TNFR2-induced and Fas results in increased susceptibility to FasL-mediated apoptosis and TNFR1-induced Fas expression is largely free of pro-apoptotic activity (**Figure 7F**). Another interaction is Fas-induced TRAIL-R1 expression (**Figure 7D**), which has no pro-apoptotic consequences and induction of proliferation in cells expressing both receptors (**Figure 7E**). These interactions define the nature of influences mediated by activation of the receptors through inductive cross-talk.

### Example 8

### Exposure of mPB cells to death ligands augments engraftment

In order to validate that the impact of death ligands is not a selective property of UCB cells, the experiments were performed with cryopreserved mobilized peripheral blood (mPB) cells. Cryopreserved mPB samples were thawed and incubated for 4 hours with 50ng/ml FasL, 20ng/ml TNF and 1,500ng/ml TRAIL. For comparative analysis, cells from same mPB units were grafted as unmanipulated (thawed) or following incubation in medium, and exposed to the ligands. A total of 10⁷ cells (without exclusion of dead cells) were transplanted into busulfan-conditioned NOD.SCID mice (as shown in figure 2A) and the bone marrow was analyzed after 12 weeks. These samples were procured by mobilization for 5 days with G-CSF and collection of mononuclear cells by pheresis. These samples were frozen with DMSO, and contained -15% dead cells after thawing. Transplantation into busulfan conditioned NOD.SCID mice (**Figure 2A**) showed markedly lower levels of donor chimerism than UCB cell transplants (**Figure 8A**). However, pretransplant incubation of the cells with FasL and TNF-α, but not in control medium alone, increased substantial the levels of human xenochimerism. Analysis of the murine marrow at 12 weeks post-transplantation revealed significant increase in human B lymphocytes in recipients of cells pretreated with FasL and TNF-α (the cells were preincubated for 4 or 16 hours with the ligands) (**Figure 8B**), which is the dominant early lineage of human cell differentiation in xenochimeras. These data demonstrate that ex vivo activation of the TNF family receptors modulates quantitative and qualitative engraftment of human hematopoictic cells mobilized into peripheral blood and cryopreserved.

### Example 9

### Exposure of mPB cells to death ligands enhances myeloid progenitor function

Additional analysis of the impact of death ligands was performed in myeloid progenitor assays in semisolid cultures. Cryopreserved mPB cells were thawed and incubated for 4 and 16 hours with death ligands (50ng/ml FasL and 20ng/ml). Cells were plated at equal numbers of total cells (without elimination of the dead cells) in methylcellulose cultures stimulated with GM-CSF, IL-3 and SCF (n=5-11) (**Figure 6A**). Brief incubation for 4 hours with FasL and TNF-α induces a small rise in number of colonies, which was markedly enhanced following incubation for 16 hours

(**Figure 9A**). Further evidence of myeloid progenitor activation was evident from increased colony size induced by TNF-α (**Figure 9B**). It is not surprising that the consequences of progenitor activation by the death ligands had differential consequences on progenitor function, as development of the precursors was influences by various environments *in vivo* and *in vitro.* These data document a tropic effect of the death ligands over the activity of myeloid progenitors mobilized to the peripheral blood.

### REFERENCES

1. Aggarwal BB. Signalling pathways of the TNF superfamily: a double-edged sword. Nat Rev Immunol. 2003;3:745-756.
2. Askenasy N, Yolcu ES, Yaniv I, Shirwan H. Fas-ligand as a double-edged immunomodulator to induce transplantation tolerance. Blood 2005; 105:1396-1404.
3. Dempsey PW, Doyle SE, He JQ, Cheng G. The signalling adaptors and pathways activated by TNF superfamily. Cytokine Growth Factor Rev 2003:14:193-209.
4. Ware CF. The TNF superfamily. Cytokine Growth Factor Rev 2003;14:181-184.
5. De Maria R, Testa U, Luchetti L, Zeuner A, Stassi G, Pelosi E, Riccioni R, Felli N, Samoggia P, Peschle C. Apoptotic role of Fas/Fas ligand system in the regulation of erythropoiesis. Blood. 1999;93:796-803.
6. Gaur U, Aggarwal BB. Regulation of proliferation, survival and apoptosis by members of the TNF superfamily. Biochem Pharmacol. 2003;66:1403-1408.
7. Testa U. Apoptotic mechanisms in the control of erythropoiesis. Leukemia. 2004;18:11176-1199.
8. Maciejewski J, Selleri C, Anderson S, Young NS. Fas antigen expression on CD34+ human marrow cells is induced by interferon gamma and tumor necrosis factor alpha and potentiates cytokine-mediated hematopoictic suppression in vitro. Blood 1995;85:3183-3190.
9. Takenaka K, Nagafuji K, Harada M, Mizuno S, Miyamoto T, Makino S, Gondo H, Okamura T, Niho Y. In vitro expansion of hematopoietic progenitor cells induces functional expression of Fas antigen (CD95). Blood. 1996;88:2871-2877.
10. Nagafuji K, Shibuya T, Harada M, Mizuno S, Takenaka K, Miyamoto T, Okamura T, Gondo H, Niho Y. Functional expression of Fas antigen (CD95) on hematopoietic progenitor cells. Blood. 1995;86:883-889.
11. Saheki K, Fujimori Y, Takemoto Y, Kakishita E. Increased expression of Fas (APO-1, CD95) on CD34+ haematopoietic progenitor cells after allogeneic bone marrow transplantation. Br J Haematol. 2000;109:447
12. Bryder D, Ramsfjell V, Dybedal I, Theilgaard-Monch K, Hogerkorp CM, Adolfsson J, Borge OJ, Jacobsen SE. Self-renewal of multipotent long-term repopulating hematopoietic stem cells is negatively regulated by Fas and tumor necrosis factor receptor activation. J Exp Med. 2001;194:941-952.
13. Young JC, Lin K, Travis M, Hansteen G, Abitorabi A, Sirenko O, Murray L, Hill B. Investigation into an engraftment defect induced by culturing primitive hematopoietic cells with cytokines. Cytotherapy. 2001 ;3(4):307-20.
14. Barcena A, Muench M, Song KS, Ohkubo T, Harrison MR. Role of CD95/Fas and its ligand in the regulation of the growth of human CD34++ CD38- fetal liver cells. Exp Hematol. 1999,27:1428-1439.
15. Josefsen D, Myklebust JH, Lynch DH, Stokke T, Blomhoff HK, Smeland EB. Fas ligand promotes cell survival of immature human bone marrow CD34+CD38- hematopoietic progenitor cells by suppressing apoptosis. Exp Hematol. 1999;27:1451-1459.
16. Dybedal 1, Yang L, Bryder D, Aastrand-Grundstrom I, Leandersson K, Jacobsen SE. Human reconstituting hematopoietic stem cells up-regulate Fas expression upon active cell cycling but remain resistant to Fas-induced suppression. Blood 2003;102:118-126.
17. Broxmeyer HE, Williams DE, Lu L, Cooper S, Anderson SL, Beyer GS, Hoffman R, Rubin BY. The suppressive influences of human tumor necrosis factors on bone marrow hematopoietic progenitor cells from normal donors and patients with leukemia: synergism of tumor necrosis factor and interferon-gamma. J Immunol 1986;136:4487-4495
18. Dybedal I, Bryder D, Fossum A, Rusten LS, Jacobsen SE. Tumor necrosis factor (TNF)-mediated activation of the p55 TNF receptor negatively regulates maintenance of cycling reconstituting human hematopoietic stem cells. Blood. 2001;98:1782-1791.
19. Jacobsen FW, Rothe M, Rusten L, Gocddel DV, Smeland EB, Vciby OP, Slørdal L, Jacobsen SE. Role of the 75-kDa tumor necrosis factor receptor: inhibition of early hematopoiesis. Proc Natl Acad Sci USA 1994;91:10695-10699.
20. Zhang Y, Harada A, Bluethmann H, Wang JB, Nakao S, Mukaida N, Matsushima K. Tumor necrosis factor (TNF) is a physiologic regulator of hematopoietic progenitor cells: increase of early hematopoietic progenitor cells in TNF receptor p55-deficient mice in vivo and potent inhibition of progenitor cell proliferation by TNF alpha in vitro. Blood. 1995;86:2930-2937
21. Caux C, Favre C, Saeland S, Duvert V, Durand I, Mannoni P, Banchereau J. Potentiation of early hematopoiesis by tumor necrosis factor-alpha is followed by inhibition of granulopoietic differentiation and proliferation. Blood. 1991;78:635-644.
22. Theus JW, Justus RS, Thcus SA. A correlation between growth rate, apoptosis, tumor necrosis factor-alpha in umbilical cord blood cells infected with two strains of Mycobacterium tuberculosis. Transfusion 2009;49:1720-1727.
23. Danet GH, Lee HW, Luongo JL, Simon MC, Bonnet DA. Dissociation between stem cell phenotype and NOD/SCID repopulating activity in human peripheral blood CD34(+) cells after ex vivo expansion. Exp Hematol 2001;29:1465-1473.
24. Gothot A, van der Loo JCM, Clapp DW, Srour EF. Cell cycle-related changes in repopulating capacity of human mobilized peripheral blood CD34 cells in non-obese diabetic/severe combined immunedeficient mice. Blood. 1998;92:2641-2649.
25. Glimm H,Oh IH, Eaves CJ. Human hematopoietic stem cells stimulated to proliferate in vitro lose engraftment potential during their S/G(2)/ M transit and do not reenter G(0). Blood. 2000;96:4185-4193.
26. Jetmore A, Plett PA, Tong X, Wolber FM, Breese R, Abonour R, Orschell-Traycoff CM, Srour EF. Homing efficiency, cell cycle kinetics, and survival of quiescent and cycling human CD34 cells transplanted into conditioned NOD/SCID recipients. Blood. 2002; 99:1585-1593.
27. Liu B, Buckley SM, Lewis ID, Goldman AI, Wagner JE, van der Loo JC. Homing defect of cultured human hematopoietic cells in the NOD/SCID mouse is mediated by Fas/CD95. Exp Hematol. 2003;31:824-832.
28. Pearl-Yafe M, Yolcu ES, Stein J, Kaplan O, Shirwan H, Yaniv I, Askenasy N. Expression of Fas and Fas-ligand in donor hematopoietic stem and progenitor cells is dissociated from the sensitivity to apoptosis. Exp Hematol 2007a;35: 1601-1612.
29. Pearl-Yafe M, Mizrahi K, Stein J, Yolcu ES, Kaplan O, Shirwan H, Yaniv I, Askenasy N. Tumor necrosis factor receptors support murine hematopoietic progenitor function in the early stages of engraftment. Stem Cells. 2010;28:1270-1280.
30. Mizrahi K, Stein J, Pearl-Yafe M, Kaplan O, Yaniv 1, Askenasy N. Regulatory functions of TRAIL in hematopoietic progenitors: human umbilical cord blood and murine bone marrow transplantation. Leukemia. 2010;24:1325-1334
31. Pearl-Yafe M, Stein J, Yolcu ES, Farkas DL, Shirwan H, Yaniv I, Askenasy N. Fas transduces dual apoptotic and trophic signals in hematopoietic progenitors. Stem Cells 2007b; 25: 3194-203
32. Mizrahi K, Stein J, Yaniv I, Kaplan O, Askenasy N. TNF-α has tropic rather than apoptotic activity in human hematopoietic progenitors: involvement of TNF receptor-1 and caspase-8. Stem Cells. 2013 Jan;31(1):156-66.
33. Askenasy N, Mizrahi K, Ash S, Askenasy EM, Yaniv I, Stein J. Depletion of naïve lymphocytes with fas ligand ex vivo prevents graft-versus-host disease without impairing T cell support of engraftment or graft-versus-tumor activity. Biol Blood Marrow Transplant. 2013 Feb;19(2):185-95.
34. Mizrahi K, Yaniv I, Stein J, Askenasy N. Apoptotic signaling through Fas and TNF receptor-1 ameliorates GvHD in mobilized peripheral blood grafts. Bone Marrow Transplant. 2013; in press
35. Wang LS, Liu HJ, Xia ZB, Broxmeyer HE, Lu L. Expression and activation of caspase-3/CPP32 in CD34(+) cord blood cells is linked to apoptosis after growth factor withdrawal. Exp Hematol 2000; 28:907-915.
36. Whartenby KA, Straley EE, Kim H, Racke F, Tanavde V, Gorski KS, Cheng L, Pardoll DM, Civin CI. Transduction of donor hematopoietic stem-progenitor cells with Fas ligand enhanced short-term engraftment in a murine model of allogeneic bone marrow transplantation. Blood. 2002;100:3147-3154.
37. Pearl-Yafe M, Yolcu ES, Stein J, Kaplan O, Yaniv I, Shirwan H, Askenasy N. Fas ligand enhances hematopoietic cell engraftment through abrogation of alloimmune responses and nonimmunogenic interactions. Stem Cells 2007c; 25: 1448-1455.

## Claims

1. An ex vivo method for obtaining a population of stem and progenitor cells (SPC) with enhanced engraftment characteristics by activation of TNF family receptors, wherein said cells are suitable for being transplanted into a recipient in need of SPC transplantation, comprising:
contacting ex vivo a biological sample obtained from a donor, said biological sample comprising a population of SPC, with at least one member of the TNF superfamily or any fragment or derivative thereof which retains the activity of the complete protein, wherein said enhanced engraftment characteristics comprise increasing myeloid, lymphoid, thrombocytic or erythroid reconstitution or activity, wherein the at least one member of the TNF super family is selected from the group consisting of Fas ligand (FasL) and tumor necrosis factor α (TNF-α), and
wherein said biological sample is umbilical cord blood (UCB) and said contacting is for between 18 hours and 48 hours; or
wherein said biological sample is mobilized peripheral blood (mPB) and said contacting is for between 3 hours and 18 hours.

2. The method according to claim 1 wherein said SPC are hematopoietic stem and progenitor cells (HSPC).

3. The method according to claim 1 wherein said TNF-α is at a concentration of between 10ng/ml and 20ng/ml, or
wherein said FasL is at a concentration of between 10ng/ml and 50ng/ml.

4. The method according to any one of the preceding claims wherein said at least one member of the TNF super family is conjugated to a surface, wherein optionally said surface is a bead, and optionally said conjugation is via a linker.

5. A population of cells for use in a method of enhancing the engraftment of stem and progenitor cells (SPC) in a recipient in need of SPC transplantation, wherein said cells are stem and progenitor cells (SPC) with enhanced engraftment characteristics wherein said population of SPC with enhanced engraftment characteristics is obtained by activation of TNF family receptors and by contacting ex vivo a biological sample obtained from a donor, said biological sample comprising a population of SPC, with at least one member of the TNF super family or any fragment or derivative thereof which retains the activity of the complete protein, wherein said enhanced engraftment characteristics comprise increasing myeloid, lymphoid, thrombocytic or erythroid reconstitution or activity, wherein the at least one member of the TNF super family is selected from the group consisting of Fas ligand (FasL) and tumor necrosis factor α (TNF-α), and wherein said biological sample is umbilical cord blood (UCB) and said contacting is for between 18 hours and 48 hours; or
wherein said biological sample is mPB and said contacting is for between 3 hours and 18 hours.

6. The population of cells for use according to claim 5 wherein said SPC are hematopoietic stem and progenitor cells (HSPC).

7. The population of cells for use according to claim 5, wherein said TNF-α is at a concentration of between 10ng/ml and 20ng/ml, or wherein said FasL is at a concentration of between 10ng/ml and 50ng/ml.

8. The population of cells for use according to any one of claims 5-7 wherein said at least one member of the TNF super family is conjugated to a surface, wherein optionally said surface is a bead, and optionally said conjugation is via a linker.

## Patentansprüche

1. *Ex vivo*-Verfahren zum Erhalten einer Population von Stamm- und Vorläuferzellen (stem and progenitor cells, SPC) mit verbesserten Transplantationseigenschaften durch Aktivierung von Rezeptoren der TNF-Familie, wobei die Zellen dafür geeignet sind, in einen Empfänger transplantiert zu werden, der eine SPC-Transplantation benötigt, umfassend:
Inkontaktbringen *ex vivo* einer biologischen Probe, die von einem Spender erhalten wurde, wobei die biologische Probe eine SPC-Population umfasst, mit mindestens einem Mitglied der TNF-Superfamilie oder einem beliebigen Fragment oder Derivat davon, das die Aktivität des gesamten Proteins behält, wobei die verbesserten Transplantationseigenschaften eine Erhöhung myeloider, lymphoider, thrombozytischer oder erythroider Neubildung oder Aktivität umfasst, wobei das mindestens eine Mitglied der TNF-Superfamilie ausgewählt ist aus der Gruppe bestehend aus Fas-Ligand (FasL) und Tumornekrosefaktor α (TNF-α), und
wobei die biologische Probe Nabelschnurblut (umbilical cord blood, UCB) ist und das Inkontaktbringen zwischen 18 Stunden und 48 Stunden andauert; oder
wobei die biologische Probe mobilisiertes peripheres Blut (mPB) ist und das Inkontaktbringen zwischen 3 Stunden und 18 Stunden andauert.

2. Verfahren nach Anspruch 1, wobei die SPC hämatopoetische Stamm- und Vorläuferzellen (HSPC) sind.

3. Verfahren nach Anspruch 1, wobei der TNF-α bei einer Konzentration zwischen 10 ng/ml und 20 ng/ml liegt, oder
wobei der FasL bei einer Konzentration zwischen 10 ng/ml und 50 ng/ml liegt.

4. Verfahren nach einem der vorangehenden Ansprüche, wobei das mindestens eine Mitglied der TNF-Superfamilie an eine Oberfläche konjugiert ist, wobei gegebenenfalls die Oberfläche ein Bead ist und gegebenenfalls die Konjugation über einen Linker erfolgt.

5. Zellpopulation zur Verwendung bei einem Verfahren zur Verbesserung der Transplantation von Stamm- und Vorläuferzellen (stem and progenitor cells, SPC) bei einem Empfänger, der eine SPC-Transplantation benötigt, wobei die Zellen Stamm- und Vorläuferzellen (SPC) mit verbesserten Transplantationseigenschaften sind, wobei die SPC-Population mit verbesserten Transplantationseigenschaften durch Aktivierung von Rezeptoren der TNF-Familie und durch Inkontaktbringen *ex vivo* einer biologischen Probe erhalten wird, die von einem Spender erhalten wurde, wobei die biologische Probe eine SPC-Population umfasst, mit mindestens einem Mitglied der TNF-Superfamilie oder einem beliebigen Fragment oder Derivat davon, das die Aktivität des gesamten Proteins behält, wobei die verbesserten Transplantationseigenschaften eine Erhöhung myeloider, lymphoider, thrombozytischer oder erythroider Neubildung oder Aktivität erhöht, wobei das mindestens eine Mitglied der TNF-Superfamilie ausgewählt ist aus der Gruppe bestehend aus Fas-Ligand (FasL) und Tumornekrosefaktor α (TNF-α), und wobei die biologische Probe Nabelschnurblut (umbilical cord blood, UCB) ist und das Inkontaktbringen zwischen 18 Stunden und 48 Stunden andauert; oder wobei die biologische Probe mPB ist und das Inkontaktbringen zwischen 3 Stunden und 18 Stunden andauert.

6. Zellpopulation zur Verwendung nach Anspruch 5, wobei die SPC hämatopoetische Stamm- oder Vorläuferzellen (HSPC) sind.

7. Zellpopulation zur Verwendung nach Anspruch 5, wobei der TNF-α bei einer Konzentration zwischen 10 ng/ml und 20 ng/ml liegt, oder
wobei der FasL bei einer Konzentration zwischen 10 ng/ml und 50 ng/ml liegt.

8. Zellpopulation zur Verwendung nach einem der Ansprüche 5 bis 7, wobei das mindestens eine Mitglied der TNF-Superfamilie an eine Oberfläche konjugiert ist, wobei gegebenenfalls die Oberfläche ein Bead ist und gegebenenfalls die Konjugation über einen Linker erfolgt.

## Revendications

1. Procédé ex vivo d'obtention d'une population de cellules souches et progénitrices (SPC) ayant des caractéristiques augmentées de greffage par activation de récepteurs de la famille des TNF, dans lequel lesdites cellules conviennent pour être greffées dans un receveur ayant besoin d'une greffe de SPC, comprenant :
la mise en contact ex vivo d'un échantillon biologique provenant d'un donneur, ledit échantillon biologique comprenant une population de SPC, avec au moins un membre de la superfamille des TNF ou l'un quelconque de ses fragments ou dérivés qui conserve l'activité de la protéine complète, dans laquelle lesdites caractéristiques augmentées de greffage comprennent un accroissement de la reconstitution ou de l'activité des myéloïdes, lymphoïdes, thrombocytes ou érythroïdes, dans laquelle l'au moins un membre de la superfamille des TNF est choisi dans le groupe constitué par le ligand Fas (FasL) et le facteur alpha nécrosant les tumeurs (TNF-α), et
dans lequel ledit échantillon biologique est du sang de cordon ombilical (UCB) et ladite mise en contact dure entre 18 heures et 48 heures ; ou
dans lequel ledit échantillon biologique est du sang périphérique mobilisé (mPB) et ladite mise en contact dure entre 3 heures et 18 heures.

2. Procédé selon la revendication 1, dans lequel lesdites SPC sont des cellules souches et progénitrices hématopoïétiques (HSPC).

3. Procédé selon la revendication 1, dans lequel ledit TNF-α est présent à une concentration comprise entre 10 ng/ml et 20 ng/ml, ou
dans lequel ledit FasL est présent à une concentration comprise entre 10 ng/ml et 50 ng/ml.

4. Procédé selon l'une quelconque des revendications précédentes, dans lequel ledit au moins un membre de la superfamille des TNF est conjugué à une surface, dans lequel optionnellement ladite surface est une bille, et optionnellement ladite conjugaison a lieu via un lieur.

5. Population de cellules pour utilisation dans un procédé pour augmenter le greffage de cellules souches et progénitrices (SPC) dans un receveur ayant besoin d'une greffe de SPC, dans laquelle lesdites cellules sont des cellules souches et progénitrices (SPC) ayant des caractéristiques augmentées de greffage, laquelle population de SPC ayant des caractéristiques augmentées de greffage est obtenue par activation de récepteurs de la famille des TNF et par mise en contact ex vivo d'un échantillon biologique obtenu auprès d'un donneur, ledit échantillon biologique comprenant une population de SPC, avec au moins un membre de la superfamille des TNF ou l'un quelconque de ses fragments ou dérivés qui conserve l'activité de la protéine complète, dans laquelle lesdites caractéristiques augmentées de greffage comprennent un accroissement de la reconstitution ou de l'activité des myéloïdes, lymphoïdes, thrombocytes ou érythroïdes, dans laquelle l'au moins un membre de la superfamille des TNF est choisi dans le groupe constitué par le ligand Fas (FasL) et le facteur alpha nécrosant les tumeurs (TNF-α), et dans laquelle ledit échantillon biologique est du sang de cordon ombilical (UCB) et ladite mise en contact dure entre 18 heures et 48 heures ; ou
dans laquelle ledit échantillon biologique est du mPB et ladite mise en contact dure entre 3 heures et 18 heures.

6. Population de cellules pour une utilisation selon la revendication 5, dans laquelle lesdites SPC sont des cellules souches et progénitrices hématopoïétiques (HSPC).

7. Population de cellules pour une utilisation selon la revendication 5, dans laquelle ledit TNF-α est présent à une concentration comprise entre 10 ng/ml et 20 ng/ml, ou
dans laquelle ledit FasL est présent à une concentration comprise entre 10 ng/ml et 50 ng/ml.

8. Population de cellules pour une utilisation selon l'une quelconque des revendications 5 à 7, dans laquelle ledit au moins un membre de la superfamille des TNF est conjugué à une surface, dans laquelle optionnellement ladite surface est une bille, et optionnellement ladite conjugaison a lieu via un lieur.
